**Europäisches Patentamt**

**European Patent Office**

**Office européen des brevets**

(19)

(11) Veröffentlichungsnummer: **0 165 336**
**A2**

(12) # EUROPÄISCHE PATENTANMELDUNG

(21) Anmeldenummer: **84109576.3**

(22) Anmeldetag: **10.08.84**

(51) Int. Cl.⁴: **A 61 N 1/42**
**A 61 N 1/40, A 47 C 31/00**

(30) Priorität: **22.05.84 DE 3419055**

(43) Veröffentlichungstag der Anmeldung:
**27.12.85 Patentblatt 85/52**

(84) Benannte Vertragsstaaten:
**AT BE CH DE FR GB IT LI LU NL SE**

(71) Anmelder: **Berner, Hans-Günter**
**Hasenholz 10**
**D-2300 Altenholz(DE)**

(72) Erfinder: **Berner, Hans-Günter**
**Hasenholz 10**
**D-2300 Altenholz(DE)**

(74) Vertreter: **Koch, Günther, Dipl.-Ing. et al,**
**Kaufingerstrasse 8**
**D-8000 München 2(DE)**

(54) **Magnetotherapeutisches Unterbett.**

(57) Bei einem magnetotherapeutischen Unterbett besteht die Oberseite aus Naturedelhaar, die Unterseite aus einem Naturgewebe und eine Einlage aus anderen natürlichen Materialien, wie Roßhaar, Kokos, Wolle oder Baumwolle. Eingebettete Magnetfolienstreifen bestehen aus einer mittleren Magnetgummischicht mit über die Länge unterschiedlich magnetisierten Permanentmagneten und zwei anliegenden Schichten, die paramagnetisches Material, beispielsweise Kupfer- und Zinkpartikel enthalten, durch die das Magnetfeld verzerrt wird und unterschiedlich gerichtete Feldvektoren gebildet werden. Die beidseitige äußere Abdeckung erfolgt durch Deckschichten, die Salze in Lösung enthalten, wodurch Elementarelemente gebildet werden. Diese Magnetfolienstreifen sind in spezieller Anordnung so eingebaut, daß ein Vektorgradient der magnetischen Induktion gebildet wird, der in bewegten Medien nach dem Faradayschen Induktionsgesetz eine Spannung induziert, die zu Strömen führt.

EP 0 165 336 A2

## Magnetotherapeutisches Unterbett

Die Erfindung bezieht sich auf ein magnetotherapeutisches Unterbett mit variablem Magnetfeld.

Die eindeutig positiven Ergebnisse der Magnetfeldtherapie auf den tierischen und menschlichen Organismus sind durch klinische Untersuchungen vielfach erwiesen. Aus bisher noch nicht restlos erforschten Ursachen ergeben sich vorteilhafte therapeutische Wirkungen auf den menschlichen Körper. Aus diesem Grunde sind zahlreiche magnetotherapeutische Geräte entwickelt worden, die sämtlich den Zweck verfolgen, ein permanentmagnetisches Feld auf bestimmte Stellen des Körpers einwirken zu lassen. So sind Magnetpflaster bekannt, die ihren Ursprung in Japan haben und gezielt an verschiedenen Stellen der Haut angebracht werden können. Ferner sind mit Magneten ausgerüstete Kleidungsstücke oder Gürtel sowie Armreifen bekannt, die gezielt die Felder, die von Permanentmagneten oder von Elementarmagneten einer Magnetfolie ausgehen, auf den menschlichen Körper einwirken lassen. Es ist ferner bekannt, Magnete oder Magnetfolien in Decken oder Unterbetten einzubauen, um so eine Person im Zustand der Entspannung, insbesondere während des Schlafes wirksam einer magnetotherapeutischen Behandlung aussetzen zu können.

Der Erfindung liegt die Aufgabe zugrunde, ein
therapeutisch wirksames Unterbett zu schaffen,
welches derart ausgebildet ist, daß die Wirkung auf
den menschlichen Körper noch weiter verbessert wird.

Gelöst wird die gestellte Aufgabe durch die im Kennzeichnungsteil des Patentanspruchs 1 angegebenen Merkmale.

Die spezielle Magnetisierung und Ausbildung der Magnetfolienstreifen ergibt sich aus den Unteransprüchen 5
bis 9.

Für die im Anspruch 2 gekennzeichneten Bedingungen
ist es biophysikalisch wichtig, daß die Polarität des
Organismus (Hannemann, Dr.Voll, Dr. Ludwig et.al) Kopf
bis Handbreite oberhalb des Knies Nordpol, und Knie bis
Füße Südpol beachtet wird. Dies geschieht durch die
exakte biologisch richtige Nord-Süd-Trennung der Magnetfolienbahnen in eine Nordpol- und eine Südpol-Ausrichtung.

Weiterhin ist es notwendig die Lage- und Ortspunkte der
wichtigsten Meridian-Fernpunkte an Waden und Füßen
(YANG-Meridiane) zu beachten und mit der richtigen
Polarität zu therapieren. Dies sind vor allem: B 60
Kunlu, B 61 PuShen, B 62 Shen Mai, B 64 JingGu, B 65
ShuKu, B 67 ZhiYin, G 37 Shen Mai, G 41 ZulinQui, G 43 Xia XI, G 44 Zu Chiaa YIN, M 41 Jie Chi, M 44 Nei
Ting, M 45 Li Tui.

Diese Meridiane haben ihre Fernpunkte an Füßen
und Waden und müssen mit dem Südpol therapiert
werden. Dieses geschieht ebenfalls durch die Südpol-
Ausrichtung des textilen magnetotherapeutischen Unterbettes vom Knie abwärts zu den Füßen. Die gegenpoligen
Energie-Fernpunkte gemäß Anspruch 3 der YIN-Meridiane
Nacken-Rückenmark und Hände werden durch die Ausrichtung der Magnetfolienbahn von Kopf bis handbreit oberhalb des Knies mit Nordpol ebenfalls richtig therapiert.

Die unter Anspruch 2 angesprochenen biophysikalischen
Gegebenheiten fernöstlicher Energietherapie decken sich
mit neurophysiooögischen Mechanismen westlicher Forschung.
Die Erkenntnisse der neurophysiologie zeigen die Möglichkeit der Magneto-Ostimulation der Haut auf verschiedene
normale und pathophysiologische Körperfunktionen, und
zwar auf sympathische Reflexe, auf die Steuerung der
Skelettmotorik, auf die Schmerzverarbeitung, auf. In
einem Hautnerv sind Tausende von Nervenfasern enthalten,
die Nachrichten über Reize an das Zentralnervensystem
(ZNS) übertragen. Man unterscheidet nach Leitungsgeschwindigkeit und Funktion der Rezeptoren. Wichtig ist,
die Rezeptoren der segmentalen Muskelgruppen, die vorwiegend am Rückenmark ansetzen, mit der sedierenden Polseite (Nordpol) zu therapieren, da die Zellmembranen der
betroffenen Nervenzellen nur über die sedierende Seite
(Nordpol) zu passieren und zu sensibilisieren sind.

Dies geschieht durch die Anordnung der Magnetfolienbahnen mit dem mittleren Streifen Ø beginnend ca.
30 cm von der Unterbett-Oberkante und ca. 80 cm Länge
entlang der Wirbelsäulenlage. Die weiteren Abstände
links und rechts von jeweils ca. 8 cm ergeben sich
aus der Skelettanatomie sowie der Lage der Haed'schen
Zonen, welche ebenfalls für die Aufnahme sedierender
magnetischer Energie sensibilisiert sind und sich
im Bereich der Grenzen des Nordpol orientierten Feldes
der Magnetfolienbahnen befindet.

Durch das Herstellungsverfahren gemäß Anspruch 4 wird
die durch normale statische Magnete und Magnetfolien
übliche Verstärkung der in der Natur vorhandenen 10 Hz-
Schumann-Wellen - die zu Mikrostreß-Situationen und zur
pathologischen Thrombozyten-Adhäsivitäts-Erhöhungen führen kann (Philips-Forschungslabor Hamburg sowie Dr. W.
Ludwig Uni Freiburg) - verhindert.

Die bevorzugte Ausführung der Magnetfolienstreifen
ergibt sich aus den Ansprüchen 5 bis 10. Dabei dient
ein statisches variables heterogenes Magnetfeld als
Informationsträger für eingelagerte Mineralstoffe.
Hierbei handelt es sich um die im Blut vorhandenen Nährsalze nach Dr. Schüssler, also insbesondere um Calzium,
Ferrum, Kalium, Magnesium, Natrium, Kupfer und Zink.
Diese Spurenelemente stehen als fluoratum, phosphoricum
und sulfurium Verbindungen zur Verfügung und bilden mit
vorgenannten Elementen die zwölf Nährsalze des Blutes.

Jedes chemische Element läßt sich physikalisch durch ein eigenes optisches Spektrum nachweisen (Kirchhoff, Bunsen). Nicht nur die verschiedenen Atome, sondern auch die Moleküle weisen charakteristische Spektren auf. Diese Objekte emittieren und absorbieren in ganz bestimmten Frequenzen magnetische Strahlung. Es ist ferner bekannt, daß das elektromagnetische Feld der Lichtwellen aus einer Metallfläche Elektronen emittieren kann, wobei es sich nicht um die Auswirkung der Intensität, sondern ausschließlich um die Wirkung der Frequenz handelt (Photoelektrische Gleichung von A. Einstein). Die dabei umgewandelte kinetische Energie nimmt proportional mit der Frequenz zu.

Diese physikalischen Grundlagen sind Ausgangspunkt der Erfindung. Licht sowie geophysikalische Strahlung, ausgedrückt als Frequenz, emittieren aus dem Magnet-Energiestreifen der Folienbahn Elektronen und transportieren diese über das Gleichfeld des mittig angeordneten statischen Magnetfeldes der Folienbahn in Form einer spezifischen Schwingung. Diese Schwingung ist aber spezifisch für die entsprechenden chemischen Moleküle der Spurenelemente im Magnet-Energiestreifen. Energie, Masse, Frequenz und Wellenzahl stehen aber durch die Konstanten h (Plancksches Wirkungsquantum) und c (Lichtgeschwindigkeit) in Zusammenhang. Es findet daher ein kurzwegiger Energietransfer statt. Dieser Energietransfer löst in biologischen Systemen Reaktionen aus. Da Zellmembranen biologischer Systeme auf kleinste Energiepotentiale reagieren, lassen sich die nachgewiesenen Reaktionen auf die Magnetfeld-Anwendung (Prof. Glombeck, Dr. Hauss und Berner, Dr.med. Russmann u.a.) physilalisch erklären.

Aus biologischer Sicht besteht eine Wechselwirkung zwischen biologischen Systemen und einer biologisch aktiven Masse. Dabei ist unzweifelhaft, daß "Biologische Systeme wechselwirken und kommunizieren über Photonen", wobei als Kopplung nur die elektromagnetische Schwingung in Frage kommen kann.

Biologische Systeme haben aber den Vorteil, die Resonanzabstimmung von $<10^1$ - $<10^{15}$ Hertz durchzuführen und benötigte Frequenzen auszufiltern. Da die Wirkung von Substanzen, also auch Medikamenten, ausschließlich von deren elektromagnetischer Schwingung abhängt - auf die Kopplung Masse-Energie-Frequenz-Wellenlänge wurde eingangs hingewiesen - ist es daher durch diese Erfindung möglich, den Informationsgehalt der eingelagerten Substanzen zu übertragen. Das Biosystem selber ist dann in der Lage, über die Resonanz das "Benötigte" herauszufiltern (Prof. Vincent, Dr.med.F. Morell).

Die gemäß der Erfindung vorgesehenen galvanischen Elemente erzeugen eine Vielzahl kleiner Ringströme, und da sie sich oberhalb der ferromagnetischen Schicht befinden, also in einem inhomogenen Magnetfeld, erzeugen sie wiederum magnetische Felder, die jedoch Wechselfelder sind, weil das Feld abhängig von der Geschwindigkeit der Cu, Zn, NaCl + $H_2O$ Elektronen ist.

In den gemäß der Erfindung vorgesehenen äußeren Schichten befinden sich die sogenannten "Schüsslerschen Nährsalze".

Durch Einwirkung der überall vorhandenen kosmischen Strahlung - auch Licht - ergibt sich der photoelektrische Effekt, der bewirkt, daß aus einer Metalloberfläche Elektronen herausgeschlagen werden. Dieser Effekt wird verstärkt durch die elektromagnetische Schwingung der inneren Schicht. Dabei ist die kinetische Energie abhängig von der Frequenz. Es wird also über die Folienbahn dem Organismus sowohl ein statisches Magnetfeld wie auch Materie bestimmter spezifischer Schwingungen zugeführt.

Nachstehend werden Ausführungsbeispiele der Erfindung anhand der Zeichnung beschrieben. In der Zeichnung zeigen

Figur 1 einen Längsschnitt einer Magnetfolienbahn zum Einbau in Streifenform in ein Unterbett gemäß Figur 5,

Figur 2 das magnetische Feld der mittleren Schicht der Bahn gemäß Figur 1, ohne Einfluß der äußeren Deckschichten,

Figur 3 eine schematische Darstellung des resultierenden Feldes der Magnetfolienbahn nach Figur 1,

Figur 4 ein Funktionsbild eines stromdurchflossenen Rings im inhomogenen Magnetfeld der äußeren Schichten der Bahn,

Figur 5 eine schematische Ansicht eines magnetotherapeutischen Unterbettes,

Figur 6 ein Deckblatt zu Figur 5, welches die relativen Lagen der Körperorgane zu den Magnetzonen des Unterbetts erkennen läßt,

Figur 7 einen Schnitt nach der Linie VII - VII gemäß Figur 5 und

Figur 8 ein Diagramm der magnetischen Flußdichte des Unterbetts.

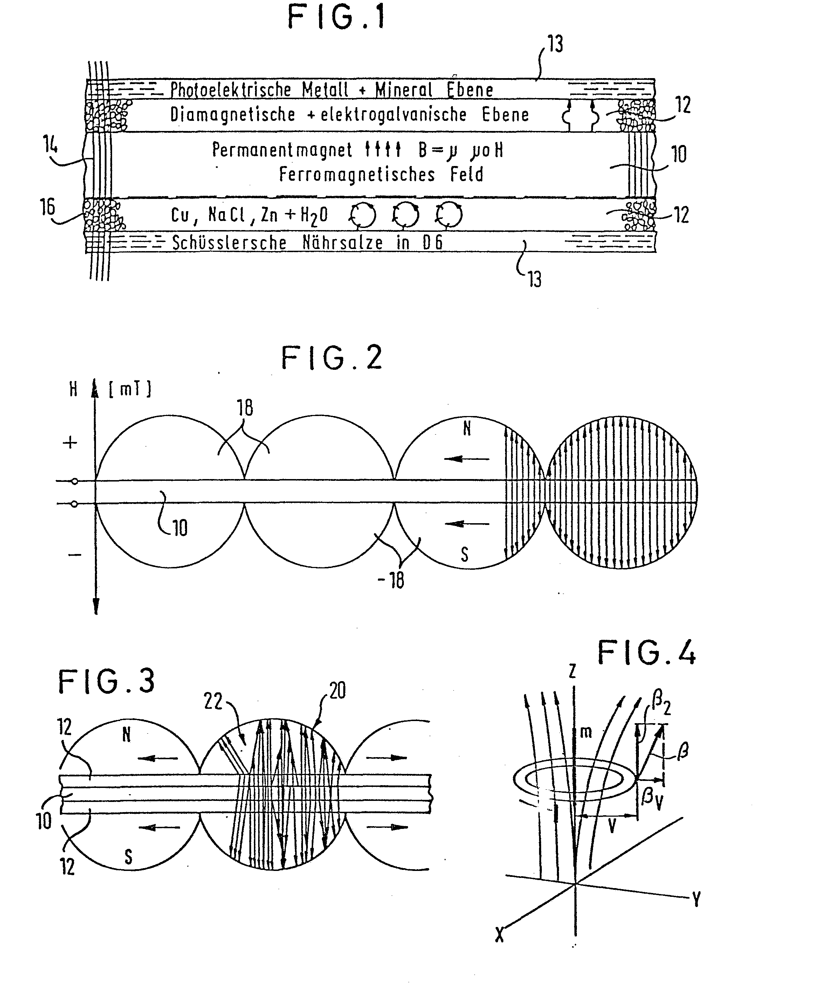

Gemäß Figur 1 besteht die Magnetfolienbahn aus mehreren Schichten 10, 12 und 13. Die mittlere Schicht 10 besteht aus "Magnetgummi", d.h. einer Schicht aus Kunststoff mit eingebetteten Ferritmagneten 14.

Die beiden der mittleren Schicht 10 beidseitig anliegenden Schichten 12 enthalten in Kautschuk gebundene diamagnetische Stoffe, vorzugsweise Partikel 16 aus Kunfer und Zink. Diese Partikel beeinflussen das von der Mittelschicht 10 ausgehende magnetische Feld.

In Figur 2 ist die magnetisierte Mittelschicht 10 dargestellt und zu beiden Seiten dieser Schicht ist die Feldstärke aufgetragen, die im dargestellten Beispiel eine Kurvenform 18, bestehend aus aneinander anschließenden Halbkreisen, aufweist. Stattdessen könnte das Feld über die Länge der Bahn (oder auch über die Breite der Bahn) sich auch sinusförmig oder nach einer anderen mathematischen Kurvenform ändern.

Figur 2 zeigt das Feld ohne Einfluß der Schichten 12.

Der Einfluß dieser Deckschichten ist in Figur 3 dargestellt. An der Stelle 20 ergibt sich eine Verstärkung, während an der Stelle 22 eine Verdünnung erfolgt. Durch Beugung der Kraftlinien infolge Verdünnung und Verdichtung durch die diamagnetischen Substanzen in den Schichten 12 ergeben sich verschiedene Austrittswinkel (Vektoren) des resultierenden Kraftfeldes. Es hat sich gezeigt, daß hierdurch besonders günstige Ergebnisse erlangt werden können.

Bei einem ausgeführten Beispiel wurde ein Magnetgummistreifen als Rollenware mit einer Dichte von 2,8 bis 3,95 g/cm$^3$ benutzt. Die Magnetisierung wurde in der Weise durchgeführt, daß sich eine Remanenz von 0,1 bis 500 mT ergab. Hierzu wurden Magnetisierungsströme zwischen 0,1 und 1800 kA eingesetzt.

Die Materialstärke der Bahn betrug 0,5 bis 4 mm.

Die äußeren Schichten 13 enthalten einen Elektrolyten in flüssiger oder vorzugsweise breiiger Konsistenz, etwa wie bei Trockenbatterien. Diese Elektrolyten, beispielsweise $NaCl + H_2O$, bilden mit dem Zink bzw. Kupfer der Schichten 12 galvanische Elemente, in Form statisch verteilter Elementarelemente. Diese Elementarelemente können auch in einer Schicht gebildet werden, indem die diamagnetische Schicht mit der Elektrolytschicht kombiniert wird. Die äußeren Schichten 13 können auch photoelektrische Metalle und/oder eine Schüsslersche Nährsalzlösung enthalten. Diese besteht aus Calziumfluoralum,

Calziumphosphoricum, Ferrumphosphoricum, Kaliumchlorentum, Kaliumphosphoricum, Kaliumsulfericum, Magnesiumphosphoricum, Natriummurialicum, Natriumphosphoricum, Natriumsulforicum, Silicea, Calziumsulforicum.

Die gebildeten Elementarelemente basieren auf dem Potentialgefälle der Spannungsreihe zwischen Kupfer und Zink. Als Elektrolyt steht in der Schicht Natriumchlorid in wäßriger Lösung zur Verfügung. Das Feld entwickelt sich nach dem System der kugelsymmetrischen Ladungsverteilung nach der Gleichung des Gauß'schen Gesetzes der Elektrostatik. Es entsteht eine Vielzahl von molekularen Ringströmen, die einen Energiestatus

$$E_p = K \frac{0,8738 \, Na^2}{a}$$

ergeben. Diese molekularen Ringströme verhalten sich wie ein stromdurchflossener Ring in einem inhomogenen Magnetfeld. Die Kraft auf einen Dipol ergibt sich nach

$$F = \pi r^2 l \frac{\delta B_2}{\delta Z} \quad (Fig. 4).$$

Für die Erfindung interessieren vor allem die daraus entstehenden Frequenzen. Diese ergeben sich aus der Umrechnungsmöglichkeit von Energie in Wellenzahl bzw. Frequenz in Energie.

Oberhalb der Schichten für die magnetische und elektrogalvanische Ebene können auf einer oder beiden Seiten noch photoelektrische Mineral-Metallebenen angeordnet sein, die auch die Schüsslerschen Nährsalze enthalten können.

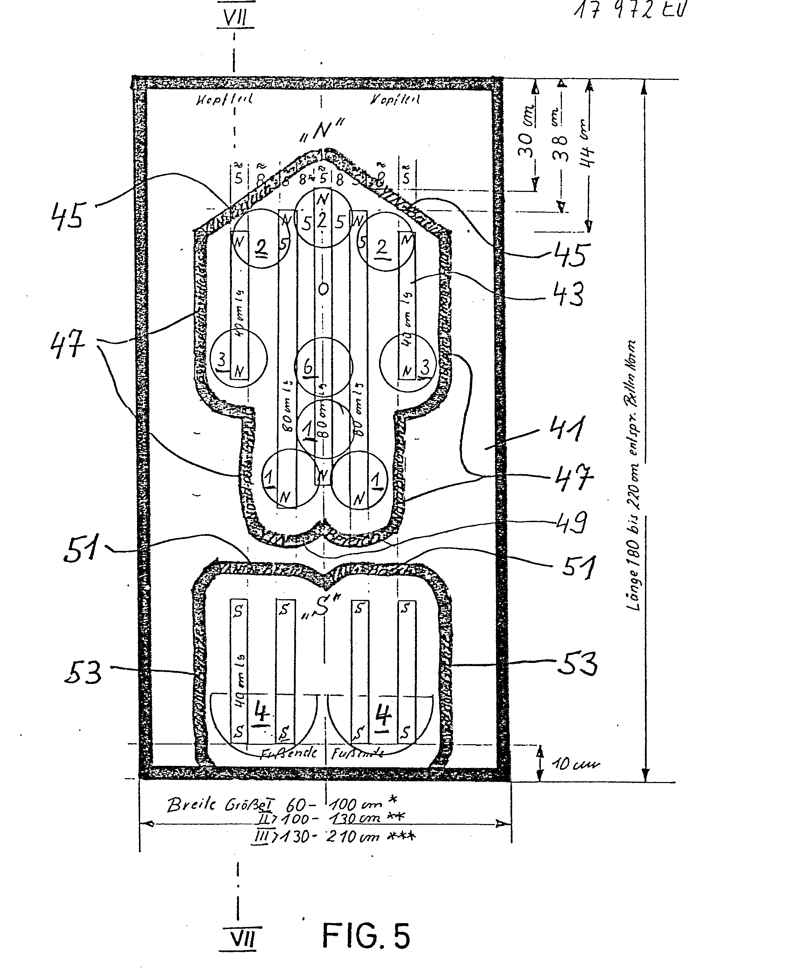

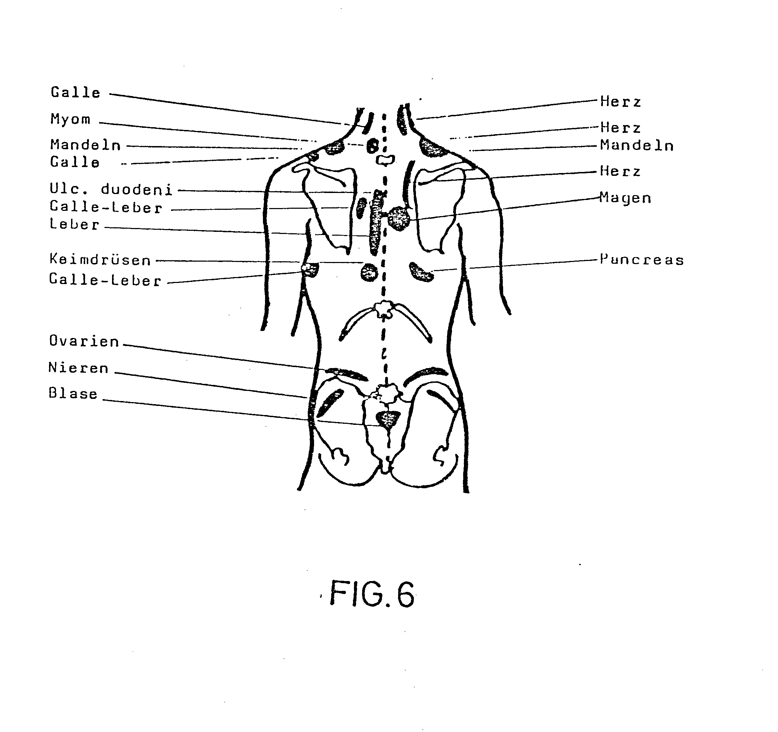

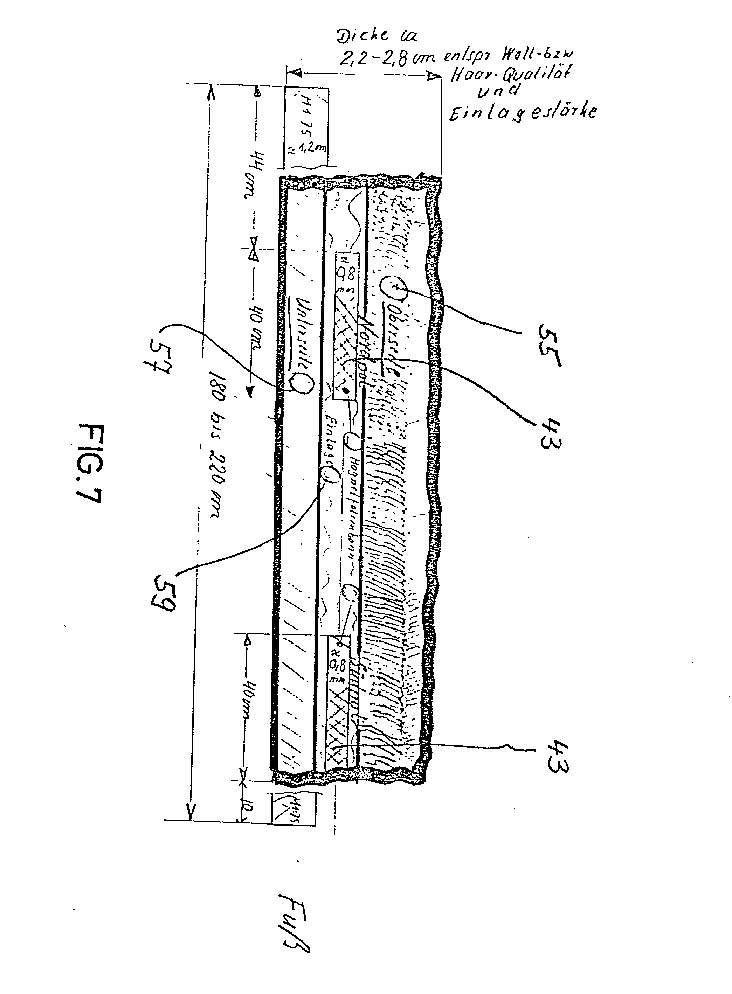

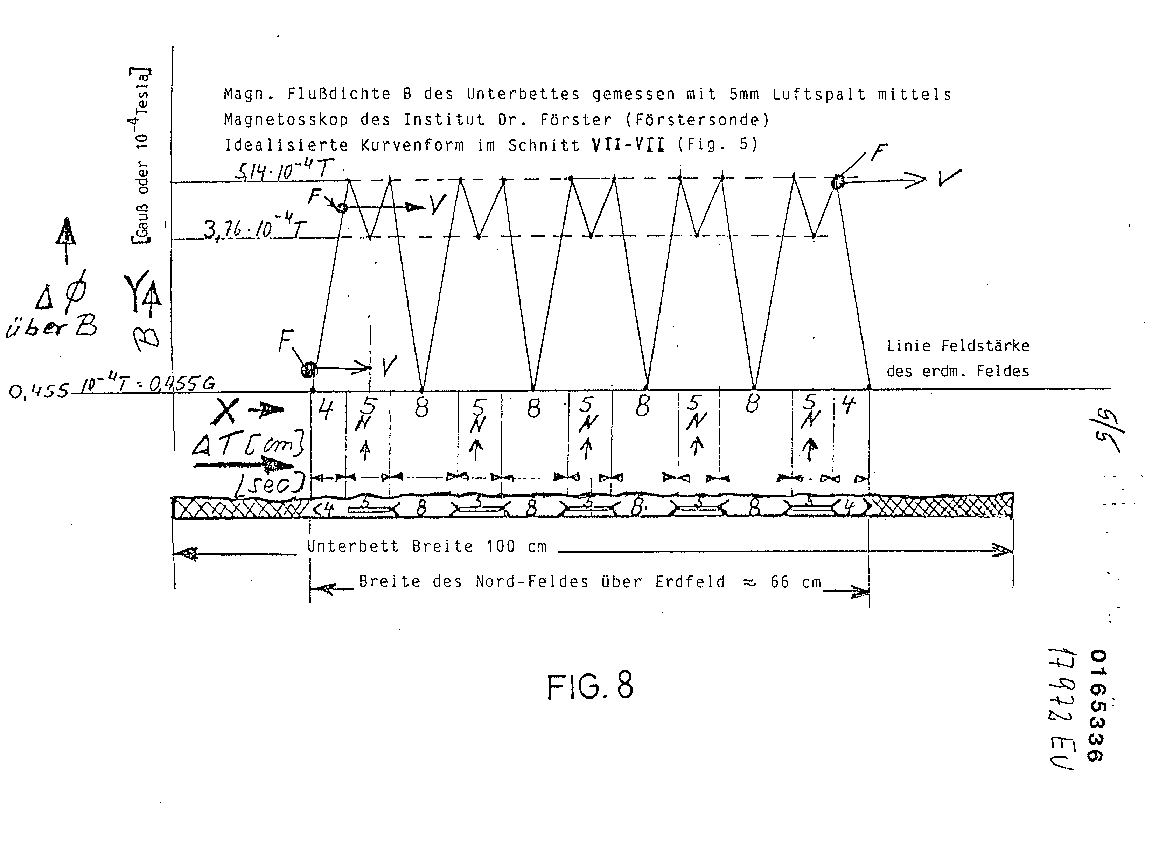

Die Figuren 5 bis 8 veranschaulichen die mögliche Anwendung einer Magnetfolienbahn gemäß Figur 1 bis 4 bei einem magnetotherapeutischen Unterbett. Die Magnetfolienbahn ist in Streifenform in spezieller Anordnung in das textile Unterbett 41 eingebettet. Sämtliche in diesem aus Naturwolle oder Naturhaar oder einer Kombination hiervon bestehenden Unterbett eingesetzten Magnetstreifen sind mit dem Bezugszeichen 43 bezeichnet. Die relative Lage der Magnetstreifen 43 im Unterbett ist für die therapeutische Wirkung entscheidend, und aus diesem Grunde ist es unentbehrlich, in der Zeichnung Maßangaben aufzunehmen, die auf unterschiedliche Größen des menschlichen Körpers bezogen sind. Die Figuren 5 und 6 lassen eine Relation zwischen den Magnetstreifen und den Haed'schen Zonen des menschlichen Körpers erkennen. In Figur 5 sind einzelne Bereiche eingezeichnet, die wie folgt charakterisiert sind:

Die Zonen 1 stellen den LWS-Bereich dar und den Bereich des Nervenzentrums des dritten bis sechsten Rückenwirbels.

Die Zonen 2 und 5 stellen den HWS-Bereich und die Haed'schen Zonen von Lunge und Herz mit der periph. Durchblutung dar.

Die Zonen 3 sind die Meridian-Endpunkte des Extr.YIN-Bereichs "Nordpol" .

Die halbkreisförmig gekennzeichneten Zonen 4 stellen die Meridian-Endpunkte des Extr.YANG-Bereichs "Südpol" dar.

Die Zone 6 stellt die Hormon-Sekret-Funktion der Pankreas-Sedirent dar.

In Fig. 5 ist das untere Ende das Fußende und das obere Ende das Kopfende. Die Polarität der Streifen ergibt sich aus den Bezeichnungen "N" bzw. "S".

In Figur 5 sind die verschiedenen Bereiche durch die geschlossenen dicken Linien wie folgt gekennzeichnet:

Die Abschnitte 45 kennzeichnen den YIN-Bereich, die Abschnitte 47 stellen die Nordpol-Grenze über dem erdmagnetischen Feld dar, und die Abschnitte 49 wiederum den YIN-Bereich.

Mit den Abschnitten 51 ist der YANG-Bereich gekennzeichnet und mit den Abschnitten 53 die Südpolgrenze.

In Figur 5 sind drei verschiedene Größenbereiche angegeben, wobei der Bereich Größe I die Standardgröße darstellt, der Bereich II die Übergröße mit proportionaler Veränderung der Folienbahnen, während III eine Doppelgröße mit symmetrischer Verdoppelung der Folienbahnen darstellt.

Die Figur 7 stellt einen Höhenschnitt dar, der der Übersichtlichkeit wegen nicht maßstäblich gezeichnet ist, wobei die Höhe der Magnetfolienbahn tatsächlich nur 0,8 bis 1 mm beträgt.

Die dem Körper zugewandte Oberseite des Unterbettes ist mit 55 bezeichnet, während die Unterseite mit 57 gekennzeichnet ist. Auf der linken Seite gemäß Fig. 7 befindet sich der Kopfteil, und auf der rechten Seite der Fußteil.

Die Figur 8 läßt die magnetische Flußdichte B des Unterbetts gemessen mit 5 mm Luftspalt erkennen. Es ist hier die idealisierte Kurvenform dargestellt.

Die Oberseite 55 des Unterbettes besteht gemäß Fig. 7 aus Naturedelhaar wie Lama, Kamelhaar, Angora u.s.w., oder Naturwolle (Merinowolle etc.), während die Unterseite 57 aus einem Naturgewebe, z.B. Baumwolle besteht. Die dazwischen befindliche Einlage 59 besteht ebenfalls aus natürlichen Materialien wie Roßhaar, Kokos, Wolle oder Baumwolle.

Durch die spezielle Magnetisierung, wie sie sich aus Figur 3 ergibt und die spezielle Anordnung der Magnetfolienstreifen, wie sie sich aus den Figuren 5 und 7 ergibt, wird ein Vektorgradient der magnetischen Induktion gebildet, der in bewegten Medien nach dem Faradayschen Induktionsgesetz eine Spannung induziert, die zu Feinströmen im bewegten Medium führt, soweit es elektrisch leitfähig ist.

Die spezielle Magnetisierung der Magnetfolienstreifen 43 und deren Anordnung (Fig. 3 bzw. Fig. 8) ergibt einen periodisch variierenden Induktionsverlauf, wovon in Figur 8 die Komponente BY gezeigt ist (x ist die Querachse des Bettes im Schnitt VII - VII gemäß Fig. 5).

In Richtung X liegt ein Vektorgradient der magnetischen Induktion vor. In einer mit der Geschwindigkeit V in X-Richtung bewegten Zelle (Blut, Lymphe) mit Querschnitt F wird laut Faradayschen Induktionsgesetz eine Grenzflächenspannung U gebildet, die sich ergibt zu

$$U = - \oiint \left( \frac{d \, B \, y}{d \, x} \times V; \, dF \right)$$

$$\text{mit } V = \frac{dx}{dt}$$

Hierbei bedeutet die Klammer das Innere (skalare) Produkt aus dem dyadischen Produkt (Induktionsgradient mal Geschwindigkeit) = $\frac{dB}{dt}$ und dem Flächendifferential dF.

Die in den Zellen gebildete Grenzflächenspannung ist also dem Induktionsgradienten grad By = $\frac{d \, By}{dx}$ nach Fig. 4, der Zellengeschwindigkeit V und dem Querschnitt der Zellen proportional, bei der elektrischen Leitfähigkeit der Zellen (Blut, Lymphe) bildet sich dadurch ein Feinstrom aus.

Patentansprüche:

1. Magnetotherapeutisches Unterbett mit statischem variablem Magnetfeld, dadurch gekennzeichnet, daß es aus reinen Naturprodukten und einem Magnetfolienstreifen (43) gemäß Figur 1 besteht, daß die Oberseite (55) aus Naturedelhaar (Lama, Kamelhaar, Angora etc.) oder Naturwolle (Merinowolle etc.) besteht, daß die Unterseite (57) aus einem Naturgewebe wie z.B. Baumwolle besteht, daß die Einlage (59) ebenfalls aus natürlichen Materialien wie Roßhaar, Kokos, Wolle oder Baumwolle besteht, daß die Magnetfolienstreifen (43) aus einer Fünfschichtenbahn besteht, die eine mittig angeordnete ferromagnetische Schicht (10) mit in einer Dimension und Richtung sich erhöhender und erniedrigender Flußdichte (Fig. 2), eine darüber und darunter angeordnete diamagnetisch und elektrogalvanisch wirkende Ebene (12) (Fig.1) sowie eine darüber und darunter liegende photoelektrische Metall- und Mineralebene (13) aufweist, in welcher u.a. die zwölf Schüsslerschen Nährsalze enthalten sind, und daß durch spezielle Magnetisierung (Fig. 3) und spezielle Anordnung der Magnetfolienstreifen (Fig. 5) ein Vektorgradient der magnetischen Induktion gebildet wird (Fig. 8), der in bewegten Medien nach dem Faradayschen Induktionsgesetz eine Spannung induziert, die zu Feinströmen im bewegten Medium führt, soweit es elektrisch leitfähig ist.

2. Magnetotherapeutisches Unterbett nach Anspruch 1, dadurch gekennzeichnet, daß die magnetotherapeutischen Magnetfolienstreifen (43) so angeordnet sind, daß die biophysikalischen und neurophysiologischen Anforderungen erfüllt sind (Fig. 5).

3. Magnetotherapeutisches Unterbett nach Anspruch 1 oder 2, dadurch gekennzeichnet, daß die gegenpoligen Meridiane und Zonen getrennt therapiert sind.

4. Verfahren zur Herstellung eines magnetotherapeutischen Unterbettes nach einem der Ansprüche 1 bis 3, dadurch gekennzeichnet, daß unter Einschluß einer Solitonen-Dynamik eine Vielzahl "180°-Blochwände" gebildet wird, indem die Moleküle der in der Folienschicht (12) (Fig. 1) gelösten Substanzen zusammen mit den $H_2O$-Molekülen sogenannte "$\widetilde{\pi}$-Kinks" bilden, wobei Energie dieser "Kinks" proportional zur Fläche der Grenzflächen der Cu-Zn-$H_2O$-Moleküle der Schicht (12) und zur Bindungsenergie der cluster stabilisierenden Bindungen zwischen den beteiligten $H_2O$-Molekülen ist.

5. Magnetotherapeutisches Unterbett nach einem der Ansprüche 1 bis 3, dadurch gekennzeichnet, daß in den Magnetfolienstreifen (43) die Magnetpartikel in einer Richtung polarisiert und in unterschiedlicher Stärke magnetisiert sind, und daß mit dem Magnetfeld ein elektrisches Gleichfeld kombiniert ist, wobei galvanische Elementarelemente, deren Metalle (beispielsweise Zn,Cu)
in einer Schicht (12) und der Elektrolyt in
einer anderen Schicht (13) enthalten sind,
gebildet werden.

6. Magnetotherapeutisches Unterbett nach Anspruch 5,
dadurch gekennzeichnet, daß sie als Verbundkörper aus fünf Schichten (10, 12, 13) besteht,
von denen nur die mittlere Schicht (10)
Permanentmagnetpartikel (14) enthält, daß die
mit den Magnetpartikeln (14) versehene flexible
Kunststoffmatrixfolie (10) beidseitig durch eine
flexible diamagnetische und elektrogalvanisch
wirkende Schicht (12) abgedeckt ist und daß die
beiden Schichten (12) in einer Matrix diamagnetische
Partikel (16) eingebettet tragen, die mit einem
Elektrolyten( beispielsweise $NaCl + H_2O$) in den
äußeren Schichten (13) die galvanischen Elementarelemente bilden, wobei die diamagnetischen Partikel (16) aus Kupfer und Zink bestehen und der
Elektrolyt aus Natriumchlorid und Wasser.

7. Magnetotherapeutisches Unterbett nach den
Ansprüchen 5 oder 6,
dadurch gekennzeichnet, daß eine Vielzahl kleiner
galvanischer Elemente innerhalb der Schichten der
Magnetstreifen (43) vorhanden ist.

8. Magnetotherapeutisches Unterbett nach
Anspruch 5 oder 6,
dadurch gekennzeichnet, daß sich oberhalb
der diamagnetischen und elektrogalvanischen
Ebene auf jeder Seite noch eine photoelektrische Mineral-Metallebene befindet.

9. Magnetotherapeutisches Unterbett nach
Anspruch 8,
dadurch gekennzeichnet, daß diese Schicht aus
den "Schüsslerschen Nährsalzen" in der
Potenzierung D6 besteht.

10. Magnetotherapeutisches Unterbett nach Anspruch 7,
dadurch gekennzeichnet, daß die Elemente eine
Vielzahl von molekularen Ringströmen bilden,
die einen Energiestatus

$$E_p = K \frac{0,9738 \ Na^2}{a}$$

ergeben.

# FIG.1

| | |
|---|---|
| Photoelektrische Metall + Mineral Ebene | |
| Diamagnetische + elektrogalvanische Ebene | |
| Permanentmagnet ↑↑↑↑ B = μ μo H | |
| Ferromagnetisches Feld | |
| Cu, NaCl, Zn + H₂O | |
| Schüsslersche Nährsalze in D6 | |

# FIG.2

# FIG.3

# FIG.4

VII

Kopfteil   Kopfteil

"N"

45

45

43

47

41

47

49

51

51

53

53

"S"

Fußende   Fußende

30 cm

38 cm

44 cm

Länge 180 bis 220 cm entspr. Betten Norm

10 cm

Breite Größe T  60 - 100 cm *
II 100 - 130 cm **
III >130 - 210 cm ***

VII

FIG. 5

17972 EU

FIG.6

4/5

Kopf

Dicke a
2,2-2,8 cm entspr Woll-bzw
Haar-Qualität
und
Einlagestärke

55   43

43

Oberseite

Nordpol

≈0,8 mm    Magnetfolienbann    Südpol

Einlage    ≈0,8 mm

M 1 75    ≈1,2m

Unterseite

M 1:75

Fuß

44 cm    40 cm    40 cm    10

180 bis 220 cm

57    59

## FIG.7

Magn. Flußdichte B des Unterbettes gemessen mit 5mm Luftspalt mittels
Magnetosskop des Institut Dr. Förster (Förstersonde)
Idealisierte Kurvenform im Schnitt VII-VII (Fig. 5)

[Gauß oder $10^{-4}$ Tesla]

$5,14 \cdot 10^{-4} T$

$3,76 \cdot 10^{-4} T$

$\Delta \phi$
über $B$

$B$

$0,455 \cdot 10^{-4} T = 0,455 G$

$F \longrightarrow V$

Linie Feldstärke
des erdm. Feldes

$X \longrightarrow$

$\Delta T$ [cm]

[sec]

4  5N  8  5N  8  5N  8  5N  8  5N  4

Unterbett Breite 100 cm

Breite des Nord-Feldes über Erdfeld $\approx$ 66 cm

FIG. 8

0165336
17972 EU